# EUROPEAN PATENT APPLICATION

(11) **EP 1 666 056 A1**
(43) Date of publication of application: **07.06.2006**
(21) Application number: 04078302.9
(22) Date of filing: 06.12.2004
(51) Int. Cl.: A61K 39/00, A61K 9/127, A61P 37/00

(54) **Cancer vaccine vesicles**

(71) Applicant: Bestewil Holding B.V., 1017 BB Amsterdam (NL)
(72) Inventor: Stegmann, Toon J.H., 2224 AA Katwijk (NL)
(74) Representative: van Westenbrugge, Andries

(57) **Abstract**

The present invention relates to methods for producing a membranous vesicle with a diameter at least 25 nm in and a lipid bilayer which comprises a viral fusion protein and tumour cell membrane components that are capable of eliciting an antitumour response. Preferably the lipid bilayer of the vesicles further comprises an amphiphilic adjuvant. The invention further relates to membranous vesicles obtainable in the methods of the invention, pharmaceutical composition comprising such vesicles as well as methods for immunisation against tumours in which the vesicle are used.

## Description

### Field of the invention

The invention relates to vaccines directed against antigens such as membrane proteins, lipids and other amphiphilic or hydrophobic molecules from tumour cells. The invention further relates to methods of preparing vesicles, comprising a lipid bilayer containing these components from tumour cell membranes, containing viral proteins with membrane fusion activity, the natural lipids of a virus, which may additionally contain amphiphilic adjuvants, to pharmaceutical compositions comprising such membranous vesicles, and their use in vaccination and cancer therapy.

### Background of the invention

One third of the people currently living in developed countries will be diagnosed with cancer at least once in their lifetime, and cancer is their second leading cause of death. Current options for treatment are radio- and chemotherapy and surgery. Vaccines for cancer are possible in principle, and the first such vaccines are entering the market (King, 2004). Most of these vaccines are therapeutic vaccines, given to patients to cure their cancer, but prophylactic vaccines are also possible. The crucial difference between classical vaccines against viruses or bacteria and cancer vaccines is that cancer cells are derived from the patient's own body, and thus present antigens, such as proteins, lipids and carbohydrates, that are not foreign to the body. The immune system tolerates the presence of such antigens, and will not mount a strong immune response to them.

Because of this immune tolerance, upon injection of a cancer vaccine, most cancer antigens are found to induce only a weak response compared to viral and bacterial antigens, and people with an intact immune system develop cancers with only a slightly lower frequency than immunosuppressed patients. Therefore, the most important challenge in cancer vaccine design is to find a way of inducing a strong immune response that breaks immune tolerance.

While classical vaccines against pathogens such as viruses contain a few purified antigens, most cancer vaccines that have shown clinical promise currently are preparations of killed or otherwise altered cancer cells. A growing number of cancer antigens, which may be proteins or other molecules, such as gangliosides, present in or on cells found in tumours, are being characterised. These antigens are often present on the plasma membrane of these cells. It is becoming clear that even for a well-defined type of cancer, important person-to-person differences in the pattern of cancer antigens associated with the tumour cells are present. Moreover, like pathogens, cancer cells can evolve through mutation in the course of the disease as a result of selection pressure, presenting different antigens, allowing them to escape protection by the immune system. Furthermore, different antigens are present during different stages of the disease. Perhaps therefore, the success of vaccines containing one or a few highly purified cancer antigens has been limited.

Two types of whole cell-based cancer vaccines have shown some potential in clinical trials: allogeneic vaccines, made of cell lines grown in a laboratory, resembling the tumour cells of a patient, but not genetically matched to the patient, and autologous vaccines, made of a patient's own cells. However, whole cells as such are not very immunogenic, and the success of such vaccines is critically dependent upon the ability of an adjuvant, a substance capable of boosting the immune response, added to the cell preparation, and intended to break immune tolerance.

The most advanced whole cell vaccines are various melanoma vaccines, such as Melacine®, produced by Corixa. This vaccine has been approved for marketing in Canada for stage IV melanoma. Melacine® consists of lysed cells from two human melanoma lines combined with Corixa's combination of strong adjuvants. Multiple repeated injections of the vaccine are given to patients; patients that respond receive monthly maintenance injections thereafter. Overall survival is not improved by Melacine®, although the vaccine works about as well as a combination of four chemotherapeutic agents, thus improving the quality of life. Because this type of vaccine contains cells that are not genetically matched to the patient, the combination of antigens may not be ideally suited for an individual patient, and despite the large number of repeated injections, the immune response is still limited.

Autologous vaccines, prepared from material surgically removed from a patient, and used to vaccinate that patient, are a better match. In a trial of 558 patients with renal cancer, autologous vaccines were produced from the individual patients' own tumour cells grown in vitro under conditions that augment their immunogenicity. A limited but clear response was demonstrated: 70 month progression free survival rates were 72% in the vaccinated vs. 59.3% in the non-vaccinated group (Jocham et al. 2004).

Colorectal cancer vaccination with mixtures of irradiated autologous tumour cells and BCG vaccine did not lead to increased overall survival, but prolonged recurrence free survival in vaccinated patients. The best results were obtained in patients with the lowest tumour burden (Vermorken et al., 1999).

While these studies provide proof of principle for whole cell based vaccines, there are also problems. The most prominent problem with whole-cell based autologous vaccines is sterility; because the cells in the vaccine are intact, filtration steps are not possible, and because the cells are derived from patient material, they may be infected before or become infected with microorganisms during surgery and preparation. Further disadvantages of whole cell preparations are that whole cells as such are not very immunogenic, and that the preparations contain the DNA of these tumour cells.

Classical viral and bacterial vaccines comprise a preparation of the antigens of these pathogens, wholly or partially purified, and sterilised by filtration. After injection, the antigens are taken up by antigen-presenting cells such as dendritic cells or macrophages, followed by a presentation of the antigens contained in the vaccines to effector cells of the immune system. While the injection of some antigens, for example subunits of influenza virus, elicits an immune response that is sufficiently strong to protect against a later infection by the same virus, this is not the case for many others. Numerous attempts to reinforce the immune response by physical or chemical means have been undertaken. The most important principles that have emerged from such experiments are: (1) For physical stimulation, multiple copies of the antigens need to be combined in particles. Particles stimulate the immune system much better than individual subunits (Ogra et al. 2001; Janeway et al. 2001), most likely because they are taken up more efficiently by antigen-presenting cells. (2) Chemical stimulation on the other hand requires that the phagocytes or the effector cells of the immune system receive certain signals through receptors present on surface of the antigen-presenting cell, for instance through the use of adjuvants, chemical compounds that are recognised by these receptors.

Two types of particles that are potentially useful for vaccines are liposomes and virosomes, because of their size and ability to contain multiple antigens. Liposomes are spherical particles, varying in diameter from 25-500 nm, consisting of a phospholipid bilayer comprising natural or synthetic phospholipids. Virosomes are similar in size to liposomes and consists of lipid bilayers containing natural or viral lipids and viral glycoproteins. Virosomes may be reconstituted viral membranes, generally produced by extraction of membrane proteins and lipids from enveloped viruses with a detergent or a short-chain phospholipid (which may be followed by addition of lipids or adjuvants), and removal of the detergent or short-chain phospholipid from the extracted viral membrane proteins and lipids, under conditions such that characteristic lipid bilayers are formed with the proteins protruding from them (Stegmann et al. 1987, WO 2004/071492). Virosomes may also comprise membranes formed from purified viral proteins and synthetic or natural lipids, or other substances that will form a bilayer.

A particularly important characteristic of virosomes involves the preservation of the receptor-binding and membrane fusion activity of the native viral envelope. Membrane fusion allows the virosomes, after cell entry mediated by receptor binding, to fuse their membrane with that of an endosome. In antigen-presenting cells, this results in the presentation of antigens on major histocompatibility complex (MHC) molecules, inducing a cytotoxic T-cell (CTL) response. The isolation of cytotoxic T cells directed against tumour antigens from tumour-infiltrating cell populations or peripheral blood indicates that such cells play an important role in natural immune defences against cancer (Cheever et al., 1993). T cells recognise an antigen presented by antigen presenting cells such as dendritic cells. After internalisation and processing of an antigen in antigen presenting cells, processed antigen forms a complex with antigen presenting molecules such as MHC proteins on the surface of the cells, and T cells recognise the processed antigen-MHC molecule complex through the antigen-specific T-cell receptor, thereby activating the T cells and consequently triggering an immune response. CTL in particular, which recognise MHC class I molecules, are believed to play an important role in this response. MHC-restricted and tumour-specific CTLs were identified, providing strong evidence in support of T cell-mediated immune responses against human tumour cells.

This type of response is especially suitable for clearing cancer cells from the body. Preservation of receptor-binding and membrane fusion activity is therefore essential for expression of the full immunogenic properties of virosomes (Arkema 2000; Bungener 2002). This feature is the main advantage of virosomes over other particles that do not have membrane fusion activity, such as liposomes or ISCOMATRIX® based vaccines (Rimmelzwaan, G.F., et al. 2004).

Virosomes containing the recombinant, purified tumour antigen Her2-neu were used to immunise mice and shown to induce both antibody and CTL responses. Vaccination lead to measurable protection of mice against a challenge with tumour cells overexpressing this protein. Even though the virosomes did not contain any adjuvant, protection of mice was as good as with the purified protein mixed with the strong adjuvant Freund's complete adjuvant (Schwaninger, R. et al. 2004). Although these data provide proof of principle for the use of virosomes in an animal model in which the cancer cells present a single antigen that is foreign to the animal, additional antigens and additional adjuvation will be required to produce practical vaccines for use in humans. It is thus an object of the invention to provide a cancer vaccine that presents multiple antigens, without the need to purify and characterise these antigens, preferably matched as closely as possible to a patient's genetic background with the immunogenic potential of virosomes.

### Summary of the invention

The present invention provides novel means and methods that solve a number of problems and difficulties outlined above. It provides for a pharmaceutically acceptable preparation that combines the advantages of simultaneous presentation of numerous antigens without the need to characterise these antigens with that of strong stimulation of the immune system. The invention provides for a particle of more than 25 nm in diameter, comprising a lipid bilayer, a variety of antigens, such as membrane proteins and lipids, from tumour cell membranes, a membrane fusion protein of an enveloped virus, which may additionally contain an amphiphilic adjuvant, wherein said adjuvant, said fusion protein, and said antigens interact through hydrophobic interactions, and are both present with the lipid bilayer membrane of the membrane. The particles may additionally contain lipids from the envelope of a virus. The invention further provides methods for producing such particles, comprising some or all of the following steps: i) preparing an extract of tumour cells obtained by biopsy, surgical excision, or growth of cells in a laboratory, involving the use of a detergent, or a short-chain phospholipid ii) in some embodiments contacting one or more amphiphilic molecules having adjuvant activity iii) in some embodiments, contacting a purified membrane fusion protein of an enveloped virus iv) in some embodiments, contacting lipids and v) in some embodiments, dissolving an enveloped virus in a detergent or short-chain phospholipid, removing the viral genetic material and core proteins, and contacting the viral membrane material with said tumour cell extract, adjuvants, and lipids vi) removing the detergent or short chain phospholipid under conditions that allow formation of a bilayer membrane.

Moreover, the invention provides a pharmaceutical preparation comprising membranous vesicles according to the invention, a pharmaceutically acceptable carrier, as well as the use of such membranous vesicles or a pharmaceutical preparation according to the invention in therapy or prophylaxis, either by intranasal, oral or parenteral delivery.

### Description of the invention

In a first aspect, the present invention pertains to a method for producing a membranous vesicle, larger than 25 nm in diameter, formed by reconstitution of cellular membranes. Preferably, the method is a method for producing a membranous vesicle of at least 25 nm in diameter, wherein the lipid bilayer of the vesicle comprises a fusion protein form an enveloped virus, tumour cell membrane components, and optionally an amphiphilic adjuvant. Preferably, the tumour cell membrane components are capable of eliciting an anti-tumour response upon administration to a subject. The method preferably comprises the steps of: (a) solubilising the membrane components of a tumour cell with a detergent and/or a short-chain phospholipid; (b) removing non-soluble tumour cell material; (c) adding a viral fusion protein to the solubilised tumour cell membrane components; and, (d) decreasing the concentration of the detergent and/or the short-chain phospholipid under conditions that allow formation of a membranous vesicle comprising a lipid bilayer incorporating the viral fusion protein and the tumour cell membrane components.

Tumour cells may be obtained by growing known tumour cell lines in the laboratory. Examples of such lines are the human colon cancer lines LOVO or SW1116, the human lung cancer lines NCI-H1417, NCI-H1435, and NHC-H1570, or the human liver adenocarcinoma NCI-N87. Tumour cells can also be obtained by surgical excision of tumours from a patient, or from biopsies taken, for example by thick needle aspiration, from cancer patients. The excised or biopsied material can be used directly as a source of tumour cell membrane components, i.e. without further cultivation of tumour cells prior to solubilisation. The material can be snap-frozen in liquid nitrogen and stored for future vaccine preparation. Preferably, such tissue is first lysed in a hypotonic buffer, e.g. by adding a tenfold volume of buffer at 30, 60, 90 or 150 mosmol/l at neutral pH, and homogenized by grinding with a pestle or by other known means. More preferably material is further purified by sieving, filtrations and/or centrifugation to remove macroscopic particles. The resulting suspension is solubilised by adding a short-chain phospholipid or detergent as described herein.

Alternatively, tumour cells can be isolated from biopsies or surgically removed tumours by enzymatic digestion with collagenases or hyaluronidases, or a combination of these and other suitable enzymes, after which cells are purified from the tissue by sieving and/or purification by density gradient centrifugation. Preferably, the resulting cell suspension is transferred to tissue culture flasks, and the cells are cultivated in tissue culture medium. The cells that adhere to the tissue culture flasks after overnight incubation at 37°C are (sub)cultured to provide a source of tumour cells (Oosterwijk-Wakka et al., 2002). As tumour cells can be subcultured, in contrast to normal cells, subculture provides a means for removing non-tumour cells from the preparation. In a preferred embodiment however, a single solid tumour, preferably an encapsulated tumour, is removed from the patients and solubilised, preferably without prior cultivation, using any of the means described herein for recovery of its membrane components.

The membrane components of the tumour cell are preferably solubilised using a solution of a suitable detergent or short-chain phospholipid. Suitable detergents are detergents that efficiently dissolve the cellular membrane components, but that do not denature the membrane proteins and other membrane components. Examples of such suitable detergents include e.g. zwitterionic detergents such as octaethyleneglycol mono-*N*-dodecylether. Short-chain phospholipids that are suitable for solubilisation of tumour cell membrane components in the method of the invention preferably contain acyl chains with less than twelve carbon atoms each. In a preferred embodiment the short-chain phospholipid is a phosphatidylcholine. More preferably the short-chain phospholipid is 1,2-diheptanoyl-*sn*-phosphatidylcholine (DHPC) or 1,2-dicaproyl-*sn-*phosphatidylcholine (DCPC). The short-chain phospholipids for use in the methods of the invention may be produced synthetically or semi-synthetically. Preferred concentrations of the detergents and/or short-chain phospholipids that are used in the methods of the invention are in the range of 50-200 mM for octaethyleneglycol mono-N dodecylether, 25-200 mM for the CDPC, 100-200 mM for DHPC. In general, a concentration at least 50% above the critical micelle concentration is used.

In the method of the invention the non-soluble tumour cell material and/or non membranous cell material, including e.g. the cellular genetic material, is preferably removed by centrifugation, preferably by differential centrifugation. Preferably, in a first step, non-soluble tumour cell material and macroscopic particles are removed by centrifugation at 1.000 x g for 10 min. Subsequently, the method includes at least one ultracentrifugation step of 100,000 x g for at least 30 min: in this step, only membrane molecules, such as membrane proteins and/or lipids that are molecularly solubilised in detergent or lipid micelles remain in the supernatant. The supernatant is then used to prepare the vesicle of the invention. Alternatively, to produce a membrane preparation cells may be lysed in a hypotonic buffer, at 30, 60, 90 or 150 mosmol/l at neutral pH, and homogenised by grinding with a pestle. After removal of macroscopic particles by sieving through a fine mesh steel sieve and/or centrifugation at 1.000 x g for 10 min, and a membrane preparation is made by ultracentrifugation on a discontinuous 10/40% sucrose (w/v) gradient at 95 000 x g for at 1 hour; membranes are then harvested form the 10/40 interface, after which these are solubilised by the short-chain phospholipid or detergent. The membranes are then solubilised by a detergent or short-chain phospholipid, and may be further purified by differential centrifugation as described above.

Subsequent to the addition of the viral fusion protein to the solubilised tumour cell membrane components in step (c), in step (d) of the method of the invention the concentration of the detergent and/or the short-chain phospholipids is decreased under conditions that allow formation of a membranous vesicle comprising a lipid bilayer incorporating the viral fusion protein and the tumour cell membrane components. Optionally, the solution of the viral fusion protein and the solubilised tumour cell membrane components may be filter sterilised prior to step (d). In step (d) of the methods of the invention, the concentration of the detergent and/or the short-chain phospholipid may be decreased by dialysis, diafiltration or absorption onto hydrophobic (and/or into size exclusion) beads. The skilled person will know how to select an appropriate rate of removal of the detergent and/or phospholipid that allows reformation of the lipid bilayer membrane, wherein preferably the viral fusion protein and the tumour cell membrane components interact through hydrophobic interactions, with the interior, hydrophobic region of the bilayer membrane, and/or with each other. Hydrophobic interactions are herein understood to mean non-covalent, non-electrostatic attraction forces between hydrophobic substances that are present in an aqueous environment.

A viral fusion protein is herein understood to mean an integral membrane protein of a virus, usually an enveloped virus that, if expressed on the surface of a suitable mammalian (or avian) cell, can induce fusion of the cell, at an appropriate pH, with cells that are a natural host for the virus (see e.g. Hernandez et al., 1996). Examples of viral fusion proteins for incorporation into the membranous vesicles of the invention include the Semliki Forest virus E1 protein, the Influenza virus hemagglutinin (HA) protein, the HIV gp120/gp41 proteins, the F proteins ofparamyxoviruses. Two types of viral fusion protein induced fusion can be distinguished. The first type of fusion, such as e.g. induced by the HIV gp120/gp41 proteins, occurs at neutral pH at the surface of the targeted host cell. The second type of fusion, such as e.g. induced by the Influenza virus hemagglutinin (HA) protein, occurs upon internalisation at lower pH (5.0 - 6.5) from within the endosomal compartment of the host cell. Both types of fusion are specifically included in the present invention. A preferred viral fusion protein for incorporation into the lipid bilayer of the vesicles of the invention is a fusion protein from an influenza virus.

The capability of the membranous cancer vesicles containing viral fusion proteins of the invention to fuse with a host cell is thus dependent on the presence of an appropriate viral fusion protein. However, this capability is further dependent of the lipid composition of the bilayer of the membranous cancer vesicle, as virosomes composed of synthetic lipids and viral fusion proteins have been described in the art that are incapable of fusion. The lipid composition of the membranous cancer vesicles is thus preferably chosen such that the membranes are capable of fusion with appropriate host cells at an appropriate pH (see below herein). The capability of the membranous cancer vesicles to fuse may be assayed in an erythrocyte ghost fusion assay as e.g. described in Stegmann et al., 1986. For membranous vesicles comprising the influenza hemagglutinin, and a preparation of membrane from cancer cells, a preferred fusion activity in this assay induces the fusion of at least 5, 10, 20, or 40 % of membranous vesicle vesicles with erythrocyte ghosts after 1 minute, if 1 *µ*M virosomes is mixed with 50 *µ*M erythrocyte ghosts membrane phospholipid at a pH that is optimal for the hemagglutinin in question. A preferred fusion activity for other membranous vesicles that cannot be tested by the above assay is the fusion upon addition of the membranous vesicles to cells capable of being infected by the virus from which their fusion proteins are derived. The reconstituted membranes should fuse at least 10% of the cells that would be fused by the virus from which their fusion proteins are derived.

In a further preferred method according to the invention, in step (c) additional lipids are added to the solubilised tumour cell membrane components for incorporation into the lipid bilayer of the vesicle. Preferably, the additional lipids comprise natural lipids of a viral membrane. One preferred lipid composition that provides the membranous vesicles with fusion activity is a lipid composition that comprises natural lipids of a virus. The term "natural lipids of a virus" is herein understood to mean those lipids that are present in the membrane of a virus grown on cells, preferably mammalian, or grown on embryonated eggs. The virus preferably is a membrane-containing virus such as most enveloped viruses. Preferred viruses for use as source of natural viral lipids are influenza viruses, Semliki Forest virus, or paramyxoviruses. The natural lipids of a virus are thus preferably obtained or isolated from virus particles thus grown, as opposed to synthetic lipids. However, functionally membranous vesicles of the invention may comprise purified lipids from other sources, e.g. synthetic lipids, in addition to the natural lipids. A lipid composition for the provision of the membranous vesicles with fusion activity is thus preferably a composition that is obtained or obtainable from natural viral membranes. Lipid compositions for use in the present invention thus include compositions exclusively composed of natural lipids of a virus, compositions composed of natural lipids of a virus supplemented with lipids from other sources, as well as compositions composed of lipids from various sources, which mimic the lipid composition of a natural viral membrane.

In a preferred method of the invention, the lipid bilayer of the vesicle further comprises an amphiphilic adjuvant that is added in step (c) to the solubilised tumour cell membrane components. Adjuvants are herein intended to include any substance or compound that, when used, in combination with an antigen, to immunise a human or an animal, stimulates the immune system, thereby provoking, enhancing or facilitating the immune response against the antigen, preferably without generating a specific immune response to the adjuvant itself. Preferred adjuvants enhance the immune response against a given antigen by at least a factor of 1.5, 2, 2.5, 5, 10 or 20, as compared to the immune response generated against the antigen under the same conditions but in the absence of the adjuvant. Tests for determining the statistical average enhancement of the immune response against a given antigen as produced by an adjuvant in a group of animals or humans over a corresponding control group are available in the art. The adjuvant preferably is capable of enhancing the immune response against at least two different antigens. The adjuvant of the invention will usually be a compound that is foreign to a mammal, thereby excluding immunostimulatory compounds that are endogenous to mammals, such as e.g. interleukins, interferons and other hormones. The amphiphilic adjuvant present in the membranous vesicle must be pharmaceutically acceptable for use in humans, in contrast to e.g. Quil A^{TM}, other saponins or natural endotoxic lipopolysaccharides (LPS) from Gram-negative bacteria, which are amphiphiles with adjuvant activity that have been tested in certain settings in the art. These amphiphilic adjuvants are therefore by definition are excluded from the definition of adjuvant herein. The adjuvants to be incorporated in the functionally membranous vesicles of the invention are preferably amphiphilic adjuvants.

The term "amphiphilic adjuvant" is intended to include any adjuvant, including compounds like lipopeptides, glycolipids and peptides, having hydrophobic membrane embedded and environment oriented polar (head group) moieties and which, preferably by itself, can associate with, or more preferably integrate into lipid bilayer vesicles or micelles in water. The term also includes any amphiphilic adjuvant that is stably incorporated into lipid bilayers with its hydrophobic moiety in contact with the interior, hydrophobic region of the bilayer membrane, and its polar head group moiety oriented toward the exterior, polar surface of the membrane. However, more hydrophobic adjuvants having a less pronounced amphiphilicity, i.e. having no or only weakly polar head group moieties, but which can associate with, or integrate into lipid bilayer vesicles, are specifically not excluded from the invention. The "amphiphilic adjuvants" with adjuvant activity as used herein, thus include naturally occurring or (partly) synthetic adjuvants that are capable of forming a membranous vesicle together with the membrane components of the tumour cell and the optional additional lipids, such as natural lipids of a virus in an aqueous environment under conditions that allow the formation of a membranous vesicle.

The amphiphilic adjuvants of the invention are preferably not covalently linked to the antigens but are present together in the lipid bilayer of the reconstituted membrane. The fact that the antigens and adjuvant are not covalently linked assures that processing of the antigen and presentation of its epitopes to the immune system is essentially identical to that of the natural protein alone, thus ensuring good recognition of the protein present on the natural pathogen. On the other hand, the hydrophobic interaction of the antigens and the adjuvant with the lipid bilayer (and each other) allows for a distribution of the adjuvant and antigens over the membranes in a preparation whereby the majority of the membrane vesicles in a preparation contain both the antigen and adjuvant in a single vesicle, more preferably at least 60, 70, 80, 90, 95 or 95% of the vesicles contain both the antigen and adjuvant. The combination of antigen and adjuvant in a single membrane or vesicle allows delivery of the antigen to the antigen presenting cell that is activated by the adjuvant, thereby increasing the therapeutic and/or prophylactic efficacy of the membranous vesicles.

In a preferred embodiment of the invention the amphiphilic adjuvant comprises a ligand that is recognised by a Toll-like-receptor (TLR) present on antigen presenting cells. Various ligands recognised by TLR's are known in the art and include e.g. lipopeptides, lipopolysaccharides, peptidoglycans, liopteichoic acids, lipoproteins (from mycoplasma, mycobacteria or spirochetes), double-stranded RNA (poly I:C), unmethylated DNA, lipoarabinomannan, flagellin, CpG-containing DNA, and imidazoquinolines. Such TLR-recognised ligands may be amphiphilic adjuvants by themselves, or alternatively they may be modified into an amphiphilic adjuvant, e.g. by coupling hydrophobic compounds (see below) to a polar TLR ligand. Alternatively, the amphiphilic adjuvants may target other receptors.

A preferred amphiphilic adjuvant is a lipopeptide, which may be produced synthetically or semi-synthetically. A lipopeptide of the invention is a molecule that will usually consist of one or more (oligo)peptides covalently coupled to one or more hydrophobic compounds selected from fatty acids, lipids, ceramides, plasmalogens, alkyl or alkene chains, or sterols. Generally, lipopeptides for use in the present invention preferably comprise 3, 4, 5, 6, 7, or 8, amino acids, preferably the peptides comprise 40 - 70% amino acids that are positively charged, of which lysine and arginine are preferred, and preferably the peptides comprises one or more serines and/or cysteines. Especially preferred lipopeptides are listed in Table 1.

In another embodiment of the invention said amphiphilic adjuvant is a glycolipid. Glycolipids are lipids (or other hydrophobic compounds) covalently coupled to one or more sugars. In a highly preferred embodiment the invention provides membranous vesicles according to the invention, in which the glycolipid is a α-galactosylceramide or a phosphatidyl inositol mannoside. The terms "an α-galactosylceramide" and "a phosphatidyl inositol mannoside" are intended to include any derivative of either one. Derivatives of these molecules having adjuvant activity and that are useful in the context of the present invention are e.g. described in US 5,936,076 and in US 4,542,212, respectively. Other suitable glycolipid adjuvants for use in the invention include e.g. modified forms of endotoxic lipopolysaccharides (LPS) of Gram-negative bacteria having reduced toxicity of the Lipid A portion the LPS but retaining (part of) the adjuvant activity, as may be obtained from genetically modified Gram negative pathogens and as reviewed in WO02/09746. A modified LPS for use as amphiphilic adjuvant in the invention preferably has a modified Lipid A moiety with reduced toxicity. The toxicity of a modified LPS preferably is less than the toxicity of a corresponding wild-type LPS, more preferably the toxicity of the modified LPS is less than 90, 80, 60, 40, 20, 10, 5, 2, 1, 0.5 or. 0.2% of the toxicity of the wild-type LPS. The toxicities of wild-type and various modified LPS's with reduced toxicity may be determined in any suitable assay known in the art. A preferred assay for determining the toxicity, i.e. the biological activity of the modified LPS's is the WEHI test for TNF-alpha induction in the MM6 macrophage cell line (Espevik and Niessen, 1986, J.Immunol.Methods 95: 99-105; Ziegler-Heitbrock et al., 1988, Int.J.Cancer 41: 456-461). On the other hand, a modified LPS with reduced toxicity should still have sufficient immunostimulatory activity, i.e. adjuvant activity. The modified LPS with reduced toxicity preferably has at least 10, 20, 40, 80, 90 or 100% of the immunostimulatory activity of the corresponding wild-type LPS. The immunostimulatory activity may be determined *in vivo* in laboratory animals as described above or in the Examples herein, or *in vitro,* e.g. determining the maturation of dendritic cells stimulated by incubation with the LPS to be tested by measuring the production of at least one cytokine (e.g. one of IL12, IL10, TNF-alpha, IL6 and IL-1-beta) by the LPS-stimulated dendritic cells, or by measuring the expression of at least one costimulatory molecule (e.g. CD40 or CD86) on the LPS-stimulated dendritic cells.

In another aspect of the present invention, the amphiphilic adjuvant present in the virosome according to the invention, is a peptide, preferably an amphiphilic peptide. Peptides, in particular polar peptides, with adjuvant activity may be rendered into amphiphilic adjuvants by (covalently) linking them to a suitable hydrophobic compound (see above). Alternatively, amphiphilic peptides may comprise a hydrophobic stretch of amino acids such as a transmembrane sequence as described below. A preferred peptide comprises a sequence from the Notch ligand Jagged-1 (see Weijzen et al., 2002; Genbank accession no. AAC 52020) or a sequence from the *Staphylococcus aureus* protein A. Peptides having sequences from Jagged-1 or protein A are preferably covalently coupled to a suitable hydrophobic compound (see above) and/or comprise a transmembrane sequence (see below). The (polar) part of the Jagged-1 or protein A derived peptides that protrudes from the lipid bilayer preferably comprises no more than 3, 4, 5, 6, 7, or 8, amino acids.

In a preferred method according to the invention the tumour cell membrane components that are incorporated into the lipid bilayer of the vesicle at least comprise an integral membrane protein from the tumour cell. The tumour cell membrane components that are incorporated into the lipid bilayer of the vesicle preferably also comprise one or more of the lipids, glycolipids, gangliosides and/or peptides or any other molecules that can function as antigens, from the tumour cell or tumour cell membrane. The membrane components from tumour cells that are part of the membrane of the vesicle according to the invention preferably have a hydrophobic part that is capable of being inserted in the lipid bilayer membrane of the cancer vaccine vesicle. Examples of antigens that have hydrophobic elements are integral membrane proteins, gangliosides, glycolipids, lipids, and lipopeptides. The antigens in the cancer vaccine vesicles of the present invention are oriented in the same way as they appear on the cellular membrane, but may present epitopes that are normally partially or at least temporarily hidden when present in a cellular membrane lipid bilayer. Stimulation of the immune system by these cancer vaccine vesicles may be due to a combination of their specific recognition by cells of the immune system, their particular character, the presentation of the protein, and the uncovering of hidden epitopes. Preferably, the antigens that are used in the membranous vesicles of the invention comprise one or more protective epitopes, i.e. epitopes capable of eliciting an immune response in a mammal that provides protection against a tumour expressing the antigen.

Antigens that can be applied and used in the formation of the cancer vaccine vesicles according to the invention can be derived from all sorts of tumour cells, and may comprise already characterised cancer antigens such as carcinoembryonic antigen, prostate-specific membrane antigen, prostate specific antigen, protein MZ2-E, polymorphic epithelial mucin (PEM), folate-binding-protein LK26, truncated epidermal growth factor receptor (EGRF), Thomsen-Friedenreich (T) antigen, GM-2 and GD-2 gangliosides, Ep-CAM (KSA), mucin-1, epithelial glycoprotein-2, and colon specific antigen.

Preferred protein antigens from these pathogens are integral membrane proteins. However, non-membrane protein antigens or parts thereof containing protective epitopes may also be modified for use in the present invention fusing them to a transmembrane sequence or lipid. Lipids that may be used are glycosyl phosphatidyl inositol derivatives. Fatty acids containing more than 10 carbon atoms such as palmitoyl may also be used. Transmembrane sequences or membrane-anchoring sequences are well known in the art and are based on the genetic geometry of mammalian transmembrane molecules. A transmembrane sequence usually consists of a stretch of about 10 - 30, usually around 20 amino acids, the majority of which having hydrophobic side chains. Transmembrane sequences are known for a wide variety of proteins and any of these may be used. Examples of membrane-anchoring sequences for use in the present invention include e.g. those derived from CD8, ICAM-2, IL-8R, CD4 and LFA-1. Preferably a transmembrane sequence is derived from viral integral membrane protein that is naturally present in a viral membrane. Examples thereof include the transmembrane region of human respiratory syncytial virus (RSV) glycoprotein G (e.g. amino acids 38 to 63) or the transmembrane region of influenza virus neuraminidase (e.g. amino acids 7 to 27).

Preferably, the methods of the invention for producing a membranous (cancer vaccine) vesicle comprise one or more steps of purifying the vesicles. Methods for purification vesicles are known in the art and include e.g. differential and density gradient centrifugation and/or chromatography (size exclusion-, ion exchange- and/or affinity-chromatography).

Preferably, in the methods of the invention the vesicles are produced under sterile conditions. Sterilisation may be performed subsequent or prior to each step in the method of producing the vesicles. Preferably, sterilisation is performed by filter sterilisation as is well known in the art. However, other methods of sterilisation that are compatible with the vesicles of the invention and with its components may equally be applied. Such sterilisation methods e.g. include gamma radiation. Especially preferred sterilisation of the vesicles as a final step in the production of the vesicles, preferably subsequent to purification of the vesicles.

In a further aspect, the invention relates to a method for producing a pharmaceutical composition comprising a vesicle produced or obtainable in a method of the invention wherein the method comprises the step of formulating the membranous vesicle into a pharmaceutical composition.

In a further aspect the invention relates to a membranous vesicle that is obtainable in a method according to the invention. A preferred vesicle is obtained in a method according to the invention. The vesicle preferably is larger than 25 nm in diameter and is formed by a reconstituted cellular membrane. The reconstituted membrane preferably comprises: (a) a lipid bilayer; (b) a preparation of membrane components from a tumour cell, and (c) a membrane fusion protein of a virus. In the reconstituted cellular membrane, preferably, the vial fusion protein and the membrane components from a tumour cell interact with the hydrophobic interior of the lipid bilayer, i.e. they are associated with, integrated into, and/or embedded in the bilayer of the viral membrane through hydrophobic interactions with the lipids of the bilayer and/or each other. Further preferred is that the membrane components from a tumour cell and the viral fusion protein are not covalently linked. The preparation of membrane components from a tumour cell preferably comprise all (i.e. the entire complement of) detergent- or short-chain phospholipid-soluble membrane components of a tumour cell, whereby the detergent and phospholipids and methods of solubilisation preferably are as herein described above. The membrane of the vesicle is thus preferably reconstituted from detergent- or short-chain phospholipid-solubilised membrane components of a tumour cell, a viral fusion protein and optional additional lipids as herein described. The detergent- or short-chain phospholipid-soluble membrane components of a tumour cell may include e.g. lipids, integral and other membrane proteins, gangliosides, glycolipids, and lipopeptides as they may occur in a given tumour cell's membrane.

Particularly preferred in the vesicles of the invention and methods for their production are preparations of membrane components from tumour cells that comprise all (i.e. the full complement of) membrane proteins, preferably integral membrane proteins as well as membrane associated proteins, as they naturally occur on and/or in the tumour cell's membrane.

A further preferred membranous vesicle that is produced or obtainable in a method according to the invention further comprises: (d) an amphiphilic adjuvant as described herein above.

In the membranous vesicle, preferably, the lipid bilayer has a lipid composition that is compatible with fusion, as induced by the fusion protein, of the viral membrane with a host cell of a natural host of the virus. Preferably the lipid composition is compatible with fusion in pH range that is compatible with fusion. Preferably, the fusion protein, the (optional) amphiphilic adjuvant and preferably also the preparation of membrane components from cancer cells interact with the hydrophobic interior of the lipid bilayer, i.e. are associated with, integrated into, and/or embedded in the bilayer of the viral membrane through hydrophobic interactions with the lipids of the bilayer and/or each other. Further preferred is that the fusion protein and the (optional) amphiphilic adjuvant are not covalently linked. Preferably, the (optional) amphiphilic adjuvant and the preparation of membranes from cancer cells are also not covalently linked. The viral membranes of the invention are preferably functionally membranous vesicles comprising lipids, preferably natural lipids of a virus, an (optional) amphiphilic adjuvant, a viral fusion protein and a preparation of membrane from cancer cells, wherein the (optional) amphiphilic adjuvant, lipids viral fusion proteins and antigens interact primarily through hydrophobic interactions, wherein the hydrophobic part of the amphiphilic adjuvant preferably forms an integral part of a lipid bilayer membrane, which bilayer further contains the fusion protein, antigen(s) and lipids. By functional reconstitution is meant, that the reconstituted membrane has membrane fusion activity. The vesicles of the invention may additionally comprise or be formed of synthetic or natural lipids.

In another aspect the invention relates to a pharmaceutical composition comprising a membranous vesicle obtainable or produced in a method according to the invention and a pharmaceutically acceptable carrier. Pharmaceutically acceptable stabilizing agents, osmotic agents, buffering agents, dispersing agents, and the like may also be incorporated into the pharmaceutical compositions. The preferred form depends on the intended mode of administration and therapeutic application. The pharmaceutical carrier can be any compatible, non-toxic substance suitable to deliver the membranous vesicles to the patient. Pharmaceutically acceptable carriers for intranasal delivery are exemplified by water, buffered saline solutions, glycerin, polysorbate 20, cremophor EL, and an aqueous mixture of caprylic/capric glyceride, and may be buffered to provide a neutral pH environment. Pharmaceutically acceptable carriers for parenteral delivery are exemplified by sterile buffered 0.9% NaCl or 5% glucose optionally supplemented with a 20% albumin. Preparations for parental administration must be sterile. The parental route for administration of the polypeptide or antibody is in accord with known methods, e.g. injection or infusion by intravenous, intraperitoneal, intramuscular, intraarterial, intrethecal or intralesional routes. The membranous vesicles are preferably administered by bolus injection. A typical pharmaceutical composition for intramuscular injection would be made up to contain, for example, 1 - 10 ml of phosphate buffered saline and 1 to 100 µg, preferably 15-45 µg (of antigen protein) of the membranous vesicles of the present invention. For oral administration, the active ingredient can be administered in liquid dosage forms, such as elixirs, syrups, and suspensions. Liquid dosage forms for oral administration can contain colouring and flavouring to increase patient acceptance. Methods for preparing parenterally, orally or intranasally administrable compositions are well known in the art and described in more detail in various sources, including, for example, Remington's Pharmaceutical Science (15th ed., Mack Publishing, Easton, PA, 1980) (incorporated by reference in its entirety for all purposes).

In a further aspect, the invention relates to a method for immunisation and/or vaccination against, or for prophylaxis or therapy of an infectious disease or tumour by administration of a therapeutically or prophylactically effective amount of (a pharmaceutical composition comprising) membranous vesicles of the invention to a subject in need of prophylaxis or therapy. The invention also relates to membranous vesicles of the invention for use as a medicament, preferably a medicament for vaccination against, or for prophylaxis or therapy of an infectious disease or tumour. Preferably, the method is a method is for therapeutic immunisation of subject having a tumour. In a more preferred method, the vesicles in the pharmaceutical composition comprise membrane components from the tumour of the subject. In such instances the subject may thus be treated with an autologous vaccine or immunising composition that comprises components from the subject's own tumour.

The invention further relates to the use of membranous vesicles of the invention in the manufacture of a medicament for vaccination or immunisation against, or for prophylaxis or therapy of an infectious disease or tumour. Preferably, the membranous vesicles obtainable or produced in a method according to the invention are used for the manufacture of a medicament for immunisation of a subject against a tumour. More preferably, the immunisation is a therapeutic immunisation of subject having a tumour. Most preferably, the vesicles comprise membrane components from the tumour of the subj ect.

### Description of the figures

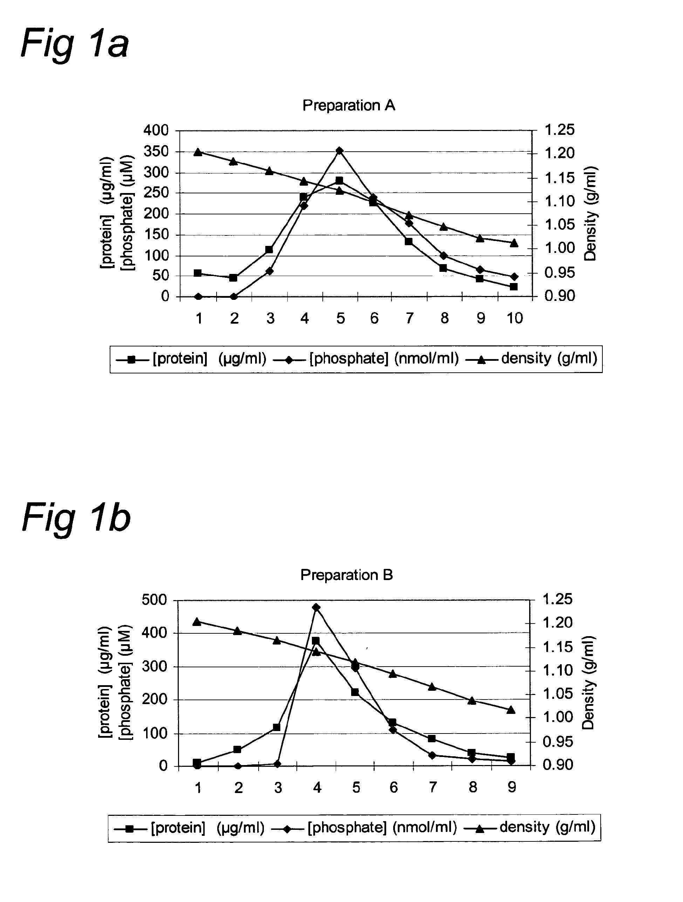

Figure 1: Equilibrium sucrose density gradient analysis of virosomes containing a membrane preparation of SW 1116 cells. A 1ml sample of preparation A (upper panel) or B (lower panel) was loaded on a linear gradient of 10-60% (w/v) sucrose in HNE-buffer and centrifuged for 24 hr at 100,000 x g at 4 °C. After centrifugation the gradient was fractionated, the density of the fractions determined by refractrometic analysis of the sucrose concentration, the protein concentration in the fractions measured by the BioRad DC protein assay, and the phospholipid concentration by phosphate analysis according to Böttcher et al. (1957).

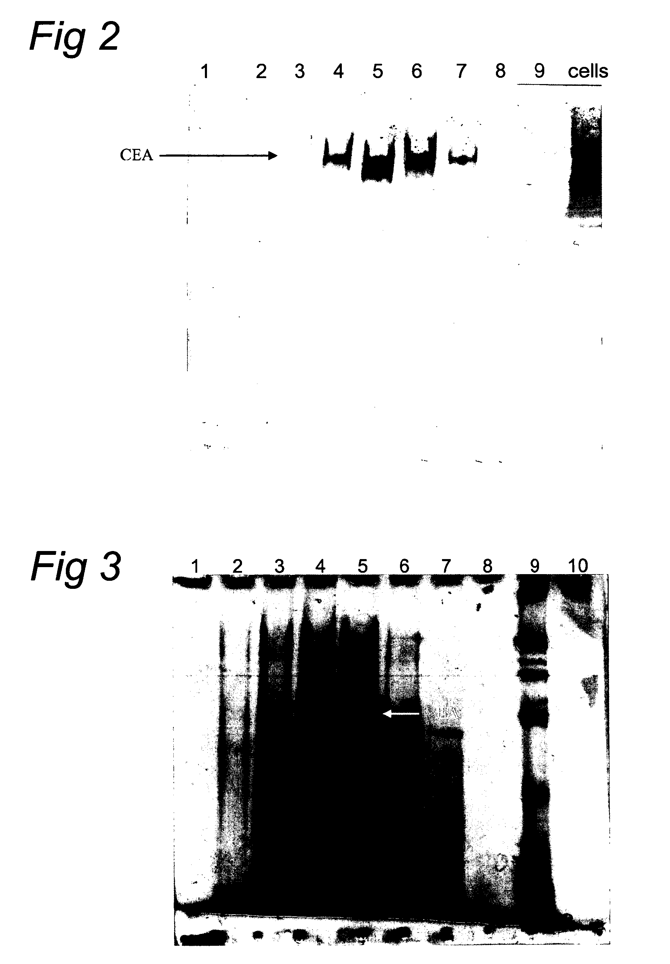

Figure 2: Western blot analysis of the fractions of preparation B (Figure 1). An SDS-PAGE gel was run under non-reducing conditions, blotted onto PVDF Immobilon-P (Millipore) according to methods known in the art, and developed using the monoclonal anti-carcinoembryonic antigen antibody C6G9 (Sigma), diluted 1: 10 000, using the secondary antibody goat-anti-mouse IgG coupled to alkaline phosphatase (Southern Biotech). Lane numbers indicate fraction numbers corresponding to those in figure 1. The band visible in fractions 1 through 9 (migrating at 180 kD) is CEA. Lane marked "cells" contains a whole cell preparation of SW 1116 cells.
Figure 3: Silver-stained SDS gel analysis of the fractions of preparation A, showing the incorporation of a variety of proteins in the virosomes. Lane numbers correspond with fractions 1-8 in figure 1, while lane 9 contains a molecular wt standard. HA indicates the position of the hemagglutinin of influenza virus.

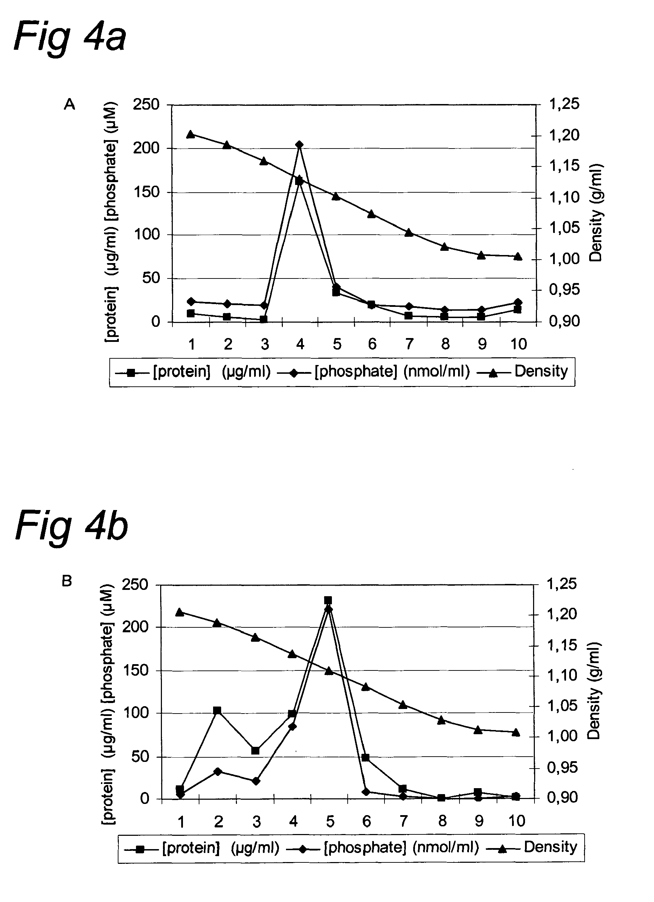

Figure 4: Equilibrium sucrose density gradient analysis of virosomes (A) containing protein and lipids from influenza, and virosomes additionally containing a membrane preparation of LOVO cells, and the lipopeptide *N*-palmitoyl-S-2,3(bispalmitoyloxy)-propyl-cysteinyl-seryl-(lysil)₃-lysine (B). A 1ml sample of either preparation was loaded on a linear gradient of 10-60% (w/v) sucrose in HNE-buffer and centrifuged for 24 hr at 100,000 x g at 4 °C. After centrifugation the gradient was fractionated, the density of the fraction was determined by refractometic analysis of the sucrose concentration, the protein concentration in the fractions measured by the BioRad DC protein assay, and the phospholipid concentration by phosphate analysis according to Böttcher et al. (1957).

### Examples

### Example 1: Production of a cancer vaccine vesicle containing the natural lipids of influenza virus, influenza membrane proteins, and a membrane preparation from the colon cancer cell ine SW 1116.

Influenza virus was produced by growing virus acquired from the World Influenza Center or the American Type Tissue Culture Collection (ATCC), using methods known to persons skilled in the art, for instance by growing the virus on embryonated eggs or cultured cells. The virus was purified, by a combination of differential and density gradient ultracentrifugation, as known in the art, and subsequently inactivated by formaldehyde according to established standard procedures.

For reconstitution by the dicaproyl-phosphatidylcholine (DCPC) method, formaldehyde-inactivated egg-grown influenza virus, strain A/Panama (9 µmol phospholipid) was first sedimented by ultracentrifugation, and subsequently resuspended with 2.1 ml HNE-buffer (30 mM Hepes pH 7.0, 100 mM NaCl, 1 mM EDTA). 2.1 ml of a solution of DCPC, 200 mM in HNE was then added. After a 30 min incubation at 0 °C to solubilise the viral membrane, the nucleocapsids were removed by centrifugation for 20 min at 100,000 x g at 4 °C. The viral membrane containing supernatant was set aside to be used for reconstitution. The whole procedure was carried out under sterile conditions in a laminar flow cabinet (class IIa).

The efficiency of solubilisation of the viral membrane was determined by analysis of the supernatant. The amount of soluble protein present in the supernatant was found to correspond closely to the amount of hemagglutinin (HA) protein, the major membrane protein that was present in the virus.

SW 1116 cells, grown for 10 days in Leibowitz' medium with 10% fetal calf serum in four 225 cm2 flasks at 37°C, were washed twice with sterile HNE buffer, and then the cells were solubilised with 8 ml of 200 mM DCPC in sterile HNE at 37°C, collected with a cell scraper, and centrifuged at 1200 x g for 10 min. The supernatant (approximately 10 ml) was mixed with a half tablet of Complete™ protease inhibitor mix containing serine, cysteine and metalloprotease inhibitors (Roche Applied Science) in 1 ml of HNE sterilised by 0,22 mm filtration, transferred to an UC TLA 100.3 ultracentrifuge tube, and spun at 100 000 g for 30 min at 4°C. The supernatant, which contained a crude plasma membrane preparation, was sterilised by filtration through a 0.22 µm cellulose acetate filter.

For reconstitution, the viral membrane supernatant and the plasma membrane preparation of the SW 1116 cells were mixed at a ratio of 1 mg of viral protein to 1 (preparation A) or 0.5 mg (preparation B) of cellular membrane proteins, as determined by the BioRad DC protein assay. The two mixtures were transferred to a 10,000 MW cut-off dialysis cassette ('Slide-A-Lyzer', Pierce Chemical Company, Rockford, IL, U.S.A.) and dialysed against 2.5 l HNE-buffer overnight, followed by six hourly changes of 2.5 l of buffer at 4 °C.

To analyse the particulate character of the reconstituted material, a sample of the dialysed material was loaded on a linear gradient of 10-60% (w/v) sucrose in HNE-buffer and centrifuged for 24 hr at 100,000xg at 4 °C. After centrifugation the gradient was fractionated, and the protein and phospholipid concentrations in the fractions were determined. A single phospholipid/protein peak was observed on both gradients, with a peak density of 1.123 g/ml for preparation A, and a peak density of 1.118 g/ml for a 1:0.5 preparation B (Figures 1 and 2). A total of 1230 µg of protein and 1258 nmol of phospholipid was recovered in 2 ml of buffer after dialysis for preparation A, and a total of 816 µg of protein and 740 µg for preparation B, in a total volume of 1.4 ml. The fact that essentially all of both phospholipids and proteins co-migrated in the direction of the bottom of the gradient as a single peak indicates that these were intimately associated. Previously it had been observed that mixtures of protein aggregates and lipid vesicles do not co-migrate in comparable gradients. These observations indicate that virosome-like-particles were formed. Since the difference in protein and lipid recovered in the virosomes is clearly linked to the amount of SW 1116 membrane preparation added to the viral membrane supernatant, these observations provide evidence for the presence of SW1116 membrane components in the virosomes.

The incorporation of cancer antigens from the plasma membrane of the SW 1116 cells was confirmed by Western Blot analysis of the fractions of these gradients (Figure 2). The peak fractions containing lipid and protein were found to contain the cancer antigen carcinoembyonic antigen (CEA), a protein normally found on the plasma membrane of SW 1116 cells, whereas the bottom and top fractions were significantly devoid of CEA. Considerable purification of the cell material in the peak fractions containing the membrane preparation, with respect to the cellular material is clearly demonstrated (cf. lane labelled "cells" in Figure 2).

The incorporation of a large variety op proteins from the cells was demonstrated by silver stained SDS page gels of the preparations (Figure 3). The peak fractions were found to contain a large variety of proteins, in addition to the influenza hemagglutinin.

### Example 2: Production of a cancer vaccine vesicle containing the lipopeptide N-palmitoyl-S-2,3(bispalmitoyloxy)-propyl-cysteinyl-seryl-(lysil)₃-lysine, the natural lipids of influenza virus, influenza membrane proteins, and a membrane preparation from the colon cancer cell line LOVO.

DCPC solubilized membrane supernatant from influenza virus strain A/Panama was prepared as in Example 1.

LOVO cells, grown in Ham's F12 medium with 10% fetal calf serum, until the cultures were 80-90% confluent, in 60 dishes of 100 mm in diameter, were washed twice with HNE buffer, and then the cells were solubilized with 4 ml of 200 mM DCPC in HNE at 37°C, collected with a cell scraper, and centrifuged at 1200 x g for 10 min. The supernatant (approximately 10 ml) was mixed with a half tablet of Complete™ protease inhibitor mix, containing serine, cysteine and metalloprotease inhibitors (Roche Applied Science) in 1 ml of HNE, sterilized by 0,22 mm filtration. The mixture was transferred to an SW 41ti ultracentrifuge tube, and spun at 100 000 g for 2 hours at 4°C. The supernatant, which contained a crude membrane preparation, was sterilised by filtration through a 0.22 µm cellulose acetate filter.

For reconstitution, 1.5 mg (protein) of viral membrane supernatant was dialyzed directly (preparation A), or the plasma membrane preparation of the LOVO cells was mixed at a ratio of 1.5 mg of viral protein to 0.5 mg of cellular membrane proteins, and 0.5 mg of *N*-palmitoyl-S-2,3(bispalmitoyloxy)-propyl-cysteinyl-seryl-(lysil)₃-lysine (preparation B). Each solution was transferred to a 10,000 MW cut-off dialysis cassette ('Slide-A-Lyzer', Pierce Chemical Company, Rockford, IL, U.S.A.) and dialysed against twice against 1.5 l HNE-buffer for 1 hour, against 1.5 l HNE-buffer overnight, and again against 1.5 l for 6 hours the next day.

To analyse the particulate character of the reconstituted material, a sample of the dialysed material was loaded on a linear gradient of 10-60% (w/v) sucrose in HNE-buffer and centrifuged for 24 hr at 100,000 x g at 4 °C. After centrifugation the gradient was fractionated, and the protein and phospholipid concentrations in the fractions were determined. A single phospholipid/protein peak was observed on both gradients, with a peak density of 1.1315 g/ml for the virosomes without lipopeptide of LOVO material (preparation A), and a peak density of 1.11 g/ml for preparation B, the virosomes with lipopeptide and LOVO material (Figure 4). A total of 585 µg of protein and 654 nmol of phospholipid was recovered in 1.7 ml of buffer after dialysis for preparation A, and a total of 1047 µg of protein and 762 µg for preparation B, in a total volume of 1.7 ml. Lipopeptides were found to be present only in the peak fractions of B. The fact that essentially all of both phospholipids and proteins co-migrated in the direction of the bottom of the gradient as a single peak indicates that these were intimately associated. Previously it had been observed that mixtures of protein aggregates and lipid vesicles do not co-migrate in comparable gradients. These observations indicate that virosome-like-particles were formed. Since the difference in protein and lipid recovered in the virosomes is clearly linked to the amount of LOVO membrane preparation added to the viral membrane supernatant, these data provide evidence for the incorporation of LOVO cell material in the virosomes.

### Example 3: Extraction of membrane components from a solid tumour, and production of virosomes containing the natural lipids of influenza virus, influenza membrane proteins, and membrane components from these tumour cells.

B16F10 melanoma cells were grown as recommended by the ATCC, and one million cells were injected subcutaneously into the right and left flank of syngeneic C57B1/6 Jola Hsd mice (Harlan). Tumours were removed 11-12 days after inoculation, at which time they reached a diameter of approximately 7 mm. The excised tumours were snap-frozen in liquid nitrogen and stored in the vapor phase of a liquid nitrogen tank. To prepare membrane components, small pieces of the tumour tissue were cut off, mixed with a tenfold volume of hypotonic buffer (20 mM NaCl, 10 mM HEPES, pH 7.4), mixed using a sterile polypropylene pestle ("Pellet pestle", Sigma). The sample was allowed to sediment for 10 min at 4°C, after which the supernatant was mixed with an equal volume of 200 mM DCPC in HNE buffer, containing a quarter tablet of Complete™ protease inhibitor mix, containing serine, cysteine and metalloprotease inhibitors (Roche Applied Science). After an incubation for 5 min, the sample was centrifuged at 800 x g for 5 min, and the supernatant was transferred to ultracentrifuge tubes, and centrifuged at 100 000 x g for 30 min at 4°C. 200 µl of the ultracentrifuge supernatant was mixed with 1.5 mg (protein) DCPC solubilized membrane supernatant from influenza virus strain A/Panama, prepared as in example 1. For reconstitution, the solution was transferred to a 10,000 MW cut-off dialysis cassette ('Slide-A-Lyzer', Pierce Chemical Company, Rockford, IL, U.S.A.) and dialysed against six times against 1 l HNE-buffer for 2 hours, and against 1.5 l HNE-buffer overnight.

### References

Arkema, A. (2000) Vaccine 18: 1327-1333

Böttcher C.J.F. et al., (1961) Anal Chim Acta 24:203-204

Bungener, L., et al. (2002) Vaccine 20: 323-338

Cheever M.A. et al., (1993) Annals N.Y. Acad. Sci. 690:101-112

Hernandez, L.D: et al., (1996) Ann. Rev. Cell Dev. Biol. 12:627-661

Janeway, C. et al. (2001) Immunobiology, 5th edition, Garland Publishing, New York

Jocham, D. et al. (2004) The Lancet 363: 594-599

King, S.E. (2004) Clin. J. Oncol. Nursing 8:278-282

Ogra PL, et al. (2001) Clin Microbiol Rev 14: 430-445

Oosterwijk-Wakka J. C. et al. (2002) J. Immunotherapy 25: 500-508

Rimmelzwaan, G.F. et al., (2004) Vaccine 22:2769-2775

Schwaninger, R. et al. (2004) Cancer Immunol. Immunother. 53: 1005-1017

Stegmann T. et al. (1987) EMBO J 6: 2651-2659

Stegmann, T.et al. (1986) J. Biol. Chem. 261:10966-10969

Vermorken, J.B. et al. (1999) The Lancet 353:345-350

Weijzen S, et al., (2002) J Immunol 169:4237-4238

**Table 1 Lipopeptides particularly suitable for making reconstituted viral membranes according to the invention.**

| |
|---|
| N-palmitoyl-S-2,3(bispalmitoyloxy)-propyl-cysteinyl-seryl-serine |
| S-2,3(bispalmitoyloxy)-propyl-cysteinyl-seryl-serine |
| N-palmitoyl-S-2,3(bispalmitoyloxy)-propyl-cysteinyl-seryl-(lysil)₃-lysine |
| S-2,3(bispalmitoyloxy)-propyl-cysteinyl-seryl-(lysil)₃-lysine |
| N-palmitoyl-S-2,3(bisoleoyloxy)-propyl-cysteinyl-seryl(lysil)₃-lysine |
| S-2,3(bisoleoyloxy)-propyl-cysteinyl-seryl-(lysil)₃ -lysine |
| N-palmitoyl-S-2,3(bismyristoyloxy)-propyl-cysteinyl-seryl-(lysil)₃-lysine |
| S-2,3(bismyristoyloxy)-propyl-cysteinyl-seryl-(lysil)₃ -lysine |
| N-palmitoyl-S-3(palmitoyloxy)-propyl-cysteinyl-seryl-(lysil)₃ -lysine |
| N-palmitoyl-S-2,3 hydroxy-propyl-cysteinyl-seryl-(lysil)₃-lysine |
| *N*-palmitoyl-S-2,3(bispalmitoyloxy)-propyl-cysteinyl-seryl-(prolyl)₃-proline |
| *N-*palmitoyl-S-2,3(bispalmitoyloxy)-propyl-cysteinyl-seryl-(glutaminyl)₃-glutaminic acid |

## Claims

1. A method for producing a membranous vesicle of at least 25 nm in diameter, wherein the lipid bilayer of the vesicle comprises tumour cell membrane components that are capable of eliciting an anti-tumour response and a viral fusion protein, wherein the method comprises the steps of:
(a) solubilising the membrane components of a tumour cell with a detergent and/or a short-chain phospholipid;
(b) removing non-soluble tumour cell material;
(c) adding a viral fusion protein to the solubilised tumour cell membrane components;
(d) decreasing the concentration of the detergent and/or the short-chain phospholipid under conditions that allow formation of a membranous vesicle comprising a lipid bilayer incorporating the tumour cell membrane components and the viral fusion protein.

2. A method according to claim 1, wherein the viral fusion protein is from an influenza virus.

3. A method according to any one of the preceding claims, wherein in step (c) additional lipids are added to the solubilised tumour cell membrane components for incorporation into the lipid bilayer of the vesicle.

4. A method according to claim 3, wherein the additional lipids comprise natural lipids of a viral membrane.

5. A method according to any one of the preceding claims, wherein the lipid bilayer of the vesicle further comprises an amphiphilic adjuvant that is added in step (c) to the solubilised tumour cell membrane components.

6. A method according to any one of the preceding claims, wherein the amphiphilic adjuvant is a lipopeptide, a glycolipid or a peptide.

7. A method according to any one of the preceding claims, wherein the amphiphilic adjuvant comprises a ligand for a mammalian Toll-like receptor.

8. A method according to claim 6, wherein the lipopeptide is selected from the group consisting of *N*-palmitoyl-S-2,3(bispalmitoyloxy)-propyl-cysteinyl-seryl-serine, S-2,3(bispalmitoyloxy)-propyl-cysteinyl-seryl-serine, *N*-palmitoyl-S-2,3(bispalmitoyloxy)-propyl-cysteinyl-seryl-(lysil)₃-lysine, S-2,3(bispalmitoyloxy)-propyl-cysteinyl-seryl-(lysil)₃ -lysine, *N*-palmitoyl-S-2,3(bisoleoyloxy)-propyl-cysteinyl-seryl-(lysil)3-lysine, S-2,3(bisoleoyloxy)-propyl-cysteinyl-seryl-serine-(lysil)₃ -lysine, *N*-palmitoyl-S-2,3(bismyristoyloxy)-propyl-cysteinyl-seryl-(lysil)₃₋lysine, S-2,3(bismyristoyloxy)-propyl-cysteinyl-seryl-(lysil)₃ -lysine, *N*-palmitoyl-S-3(palmitoyloxy)-propyl-cysteinyl-seryl-(lysil)₃ -lysine and *N*-palmitoyl-S-2,3 hydroxypropyl-cysteinyl-seryl-(lysil)₃-lysine, *N*-palmitoyl-S-2,3(bispalmitoyloxy)-propyl-cysteinyl-seryl-(prolyl)₃-proline, and *N*-palmitoyl-S-2,3(bispalmitoyloxy)-propyl-cysteinyl-seryl-(glutaminyl)₃-glutaminic acid.

9. A method according to claim 6, wherein the glycolipid is a phosphatidyl inositol mannoside, an alpha-galactosylceramide, or a modified lipopolysaccharide having reduced toxicity.

10. A method according to any one of the preceding claims, wherein the tumour cell membrane components incorporated into the lipid bilayer of the vesicle at least comprise an integral membrane protein from the tumour cell.

11. A method according to any one of the preceding claims, wherein the tumour cell membrane components incorporated into the lipid bilayer of the vesicle comprise one or more of glycolipids, gangliosides and peptides from the tumour cell.

12. A method according to any one of the preceding claims, wherein the method comprises a further step (e) in which the membranous vesicle are purified.

13. A method according to any one of the preceding claims, wherein the method comprises a further step (f) in which the membranous vesicle are sterilised.

14. A method for producing a pharmaceutical composition comprising a vesicle produced or obtainable in a method according to any one of claims 1 - 13, wherein the method comprises the step of formulating the membranous vesicle into a pharmaceutical composition.

15. A membranous vesicle obtainable in a method according to any one of claims 1 - 13.

16. A membranous vesicle of at least 25 nm in diameter and comprising a reconstituted cellular membrane comprising a lipid bilayer and incorporated therein (a) the detergent- or short-chain phospholipid-solubilised membrane components of a tumour cell; and (b) a membrane fusion protein of a virus.

17. A membranous vesicle according to claim 16, wherein the lipid bilayer further comprises additional lipids.

18. A membranous vesicle according to claim 17, additional lipids comprises natural lipids of a viral membrane.

19. A membranous vesicle according to any one of claims 16 - 18, wherein the lipid bilayer further incorporates an amphiphilic adjuvant as defined in any one of claims 6 - 9.

20. A pharmaceutical composition comprising a membranous vesicle obtainable in a method according to any one of claims 1 - 13 or a membranous vesicle according to any one of claims 16 - 19, and a pharmaceutically acceptable carrier.

21. A method for immunisation against, or for prophylaxis or therapy of a tumour by administration of a therapeutically or prophylactically effective amount of the membranous vesicles of claim 15 - 19 or the pharmaceutical composition of claim 20 to a subject in need of prophylaxis or therapy.

22. A method according to claim 21, wherein the method is for therapeutic immunisation of subject having a tumour.

23. A method according to claim 22, wherein the vesicles comprise membrane components from the tumour of the subject.

24. Use of the membranous vesicle of claim 15 - 19 in the manufacture of a medicament for immunisation against, or for prophylaxis or therapy of a tumour.

25. A use according to claim 24, wherein the immunisation is a therapeutic immunisation of subject having a tumour.

26. A use according to claim 25, wherein the vesicles comprise membrane components from the tumour of the subject.
